# EUROPEAN PATENT APPLICATION

(11) **EP 4 704 100 A1**
(43) Date of publication of application: **04.03.2026**
(21) Application number: 24197010.2
(22) Date of filing: 28.08.2024
(51) Int. Cl.: G16H 30/20, G16H 30/40

(54) **METHOD AND SYSTEM FOR PROVIDING POSITIONING GUIDANCE FOR MEDICAL IMAGING OF A PATIENT**

(71) Applicant: Siemens Healthineers AG, 91301 Forchheim (DE)
(72) Inventor: KOLLERATHU, Varghese, 560066 Bengalurur (IN); SINAI TALAULICAR, Radhika, 560035 Bangalore (IN); VINAY BHOMBORE, Vineet, 560078 Bengaluru (IN)
(74) Representative: Siemens Healthineers Patent Attorneys

(57) **Abstract**

The invention pertains to a method for providing positioning guidance for medical imaging of a patient, especially for mammography, after a request for a medical imaging procedure, comprising the steps:
a) acquiring a number of optical images (I) and/or image-like datasets of the patient,
b) automatically estimating a physical condition of the patient based on the content of the acquired number of images (I),
c) generating a view-list (V) comprising a number of suggested optimal views for the requested medical imaging procedure based on the estimated physical condition of the patient,
d) outputting the generated view-list (V) for image acquisition.

Furthermore, the invention describes a system and a medical imaging system.

## Description

The invention describes a method and a system for providing positioning guidance for medical imaging of a patient, especially for mammography, as well as a medical imaging system.

Inadequate patient positioning is one of the primary causes of rejection of ax X-ray image, e.g. in mammography. Positioning patients for imaging is dependent on varying factors such as body habitus such as patient height, size of breasts and limitations such as range of motion in shoulder, skeletal malformations like kyphosis, etc.

While there are standard guidelines for positioning for each projection (in mammography e.g. CC or MLO), the technician has to use his/her judgement to obtain the best possible images considering the limitations of the patient. For e.g. in a screening mammogram of a kyphosis patient, while the recommendation is for the standard CC and MLO view, the technician may have to resort to a reverse CC projection as an additional/alternate projection.

Typically, technicians rely on their knowledge/experience to position the patient. They may consult other senior technicians, wherever available. At times, one or more repeats are done, till the desired quality and coverage is achieved. In some systems, guidance is provided by means of standard references for each projection, which is typically of minimal use to a technician. If the images are not adequate, the patient has to be recalled.

In remote (Access-to-Care) areas, the technicians may not be as skilled/qualified as in the urban areas. Also, in mobile mammography, typically there is one technician who is onboard the van. There is no instant help from senior technicians, in case required and in these cases, it is all the more important to get it right in the first scan, as the next camp in that area may be only a few months or even years later.

Hence, positioning is highly dependent on the skills/experience/judgement of the technicians.

It is the object of the present invention to improve the known systems and methods and provide a method and a system for providing positioning guidance for medical imaging of a patient, especially for mammography, after a request for a medical imaging procedure and a medical imaging system, for overcoming the above described problems.

This object is achieved by a method according to claim 1, a system according to claim 7 and a medical imaging system according to claim 13.

A method according to the invention serves for providing positioning guidance for medical imaging of a patient, especially for mammography, after a request for a medical imaging procedure. It comprises the following steps:
a) acquiring a number of optical images and/or image-like datasets of the patient,
b) automatically estimating a physical condition of the patient based on the content of the acquired number of images and/or image-like datasets,
c) generating a view-list comprising a number of suggested optimal views for the requested medical imaging procedure based on the estimated physical condition of the patient,
d) outputting the generated view-list for image acquisition.

The method provides positioning guidance after a request for a medical imaging procedure and before image acquisition. It is preferred that this positioning guidance is given right before an image is acquired, i.e. at the time the patient is being positioned for imaging, e.g. for mammography, fluoroscopy, projection imaging, tomosynthesis, CT or MRI.

First, a number of optical images are recorded. This could be 2D or 3D pictures or videos from a camera. Alternatively or additionally, image-like datasets like depth maps could be recorded, e.g. using a depth camera or LIDAR based technology. These image-like datasets have the advantage that they could provide the necessary information, but are privacy preserving. These images are not used to identify the patient, but to estimate a physical condition of the patient. Thus, only such areas of the patient that have an influence on the examination should be regarded (or being visible in the images). For example, the face, could be left out or blurred.

It should be noted that the, image-like datasets could also be or comprise descriptions in form of words that are describing the physical appearance of the patient.

Now, the content of the images is used for estimating a physical condition of the patient in an automated manner. The physical condition could include skeletal malformations, medical equipment attached (e.g. like an IV line, a jejunostomy tube, etc.), injuries, size, weight, age or any other physical and visible feature of the patient that may affect positioning. This could be done by segmenting the image for the body of the patient, adding landmarks to the segmented patient and comparing distances or ratios of distances between these landmarks. In addition, there may also be segmented special means the patient uses such as wheelchairs or a plaster cast. This could be done with a machine learning model specially trained for image segmentation and possibly attaching and examining landmarks.

Based on the estimated physical condition, the view-list is generated. This view-list may only comprise one single view, e.g. in the case that physical condition of the patient allows acquiring images for the whole examination in one single view, or multiple views, in the case the physical condition of the patient or the examination require more than one view. The view(s) strongly depend on the physical condition of the patient. It could be required that (compared to a view list for a patient with an optimal physical condition), the view-list comprises different and/or more views.

The views of the view-list or the complete view-list could be chosen from multiple views or view-lists of a database. The database may be part of the system or an external database comprising the views for the view-list or already complete view-lists could be contacted.

Then, the generated view-list is outputted for image acquisition. This could be done by displaying the view-list for a technician and/or by outputting control commands for an automatic imaging system.

A system according to the invention serves for providing positioning guidance for medical imaging of a patient, especially for mammography, after a request for a medical imaging procedure. It comprises the following components:
- an optical measurement unit designed to acquire optical images or image-like datasets of a patient,
- an estimation-unit designed for automatically estimating the physical condition of the patient based on the content of the acquired number of images and/or image-like datasets,
- a view-unit designed for generating a view-list comprising a number of suggested optimal views for the requested medical imaging procedure based on the estimated physical condition of the patient,
- a data-interface designed for outputting the generated view-list for image acquisition.

The system is preferably designed for performing the method according to the invention. The function of the components of the system is described above.

Thus, the proposed solution is centered around providing positioning guidance to a technician based on the automated assessment of the physical condition (e.g. limitations) of the patient. For a practical approach, the solution should have the following features:
1. Initial assessment of the physical condition of the patient, e.g. using a depth camera or LIDAR based technology, and suggesting the optimal views to fulfil the request from the ordering physician. This should be done in a privacy preserving manner.
2. After a number of images is acquired (after any or after a predefined number or after acquiring all views), it is preferred to assess, whether the acquired views are sufficient to fulfil the request for examination. If not, it is preferred to recommend additional views. These additional views should also consider the physical condition of the patient.
3. It is preferred to graphically display the coverage of the examined body part (e.g. the breast tissue), as each view (a predefined number of views or every view) is taken.
4. It is also preferred to provide a facility for a technician to converse with the system seeking help in the context of the current patient.
5. A continuously learning system with a growing database which recommends the optimal scans is also preferred.

The main component of the invention is the suggestion of the optimal views based on patient condition and especially also characteristics. Prior to the examination, the technologist may enter information such as request details raised by the physician, age of the patient and any relevant prior information such as disability or physical limitation to name a few that is available.

The optical sensor, e.g. a depth camera or LIDAR based technology, is used to obtain images or depth maps. The captured images or depth maps are preferably inputted to an AI based algorithm that detects the person and detects keypoints or an anatomical location. This information is then preferably fed to another AI based algorithm which detects skeletal malformations such as kyphosis, scoliosis to name a few and other physical deformities or limitations. There could be used only one AI for both tasks, but the use of two Als is preferred since any AI could be optimally trained for its special task. However, any of these tasks could also be processed with a conventional algorithm.

It should be noted that in general the expressions "keypoint" and "landmark" could be used synonymously. Any use of the word "keypoint" implies also the word "landmark" and vice versa. The use of the word "keypoint" in this text should emphasize that the certain issue is especially advantageous for those entities which are used to detect physical limitations. The use of the expression "landmarks" should emphasize that the certain issue is especially advantageous for key areas in the breast such as nipple, or pectrolis muscle.

The results obtained could then be displayed to the technologist, and in the event if the results are wrong, it would be advantageous if the system may comprise a mechanism for the technologist to edit the outputs. Such user interactions could later be used to further improve the respective AI models.

The determined physical condition of a patient could be used to query a database that retrieves data from patients having physical characteristics similar to the current patient. The retrieved data preferably comprises the distinct views that are recommended to scan the whole region of interest, e.g. a breast, and highlights key indicators to the technologist, such as the length of Posterior Nipple Line in each view of a mammogram, or the set of landmarks visible in each view. A number of these views can be used for the view-list.

When the view-list is known, the examination may start.

This basic setup may preferably be supported by a diagnosis approach where it is estimated whether the acquired images are sufficient for the examination or not. This could be performed by a "diagnosis component". This component may comprise a series of AI algorithms (or conventional algorithms) performing diverse tasks ranging from determining the view of the input images, detecting landmarks, for segmenting the images, to determine a characterizing value. For example, a mammogram could be parsed for detecting Nipple, IMF or Pectoralis Muscle as landmarks, for segmenting the mammograms, e.g. into nipple and pectoralis muscle, in order to determine the PNL value as characterizing value.

Additionally, the component could comprise additional AI models (or conventional algorithms) which inspect the images. For example, a mammogram could be inspected for artifacts such as chin artifacts, hand artifacts or skin folds.

Thus, this diagnosis component could log the set of landmarks that were detected in each view and also log relevant clinical parameters such as a PNL value.

A third aspect is to determine regions of an organ of interest and suggest views to capture aspects of the organ that are yet to be scanned. For example, in the domain of mammogram, there exists a set of particular views which are recommended to best visualize a certain region of the breast. For example, the MLO view enables to capture a posterior aspect and axillary region of the breast.

In order to determine whether an obtained view (e.g. MLO in a mammogram) captures the required region, the method could check whether a set of predetermined landmarks can be determined from the scan. Additionally, it could also check whether a set of derived parameters, e.g. such as PNL in a mammogram, falls within an acceptable range.

This information is preferably derived from all the scans performed on the patient and then fused and presented to a technologist. The presented data preferably captures the set of aspects that has been covered or visualized from the obtained set of scans and also the set of aspects of the organ which are yet to be visualized. Additionally, it preferably also suggests views so as to capture aspects of the organ which yet to be visualized completely.

It is preferred to query a database for getting optimal views to better visualize an aspect of the organ. Prior to an exam, based on the physical patient data, the view-list is chosen from multiple views or view-lists stored in the database. A view-list should comprise the optimal views that are needed for an examination of a certain patient. It is however possible owing to the patient discomfort, or due to the challenges arising from the body habitus of the patient, the technologist may be unable to capture a clinically relevant scan (which captures certain key aspects of the organ). Under such a scenario, the technologist can interact with the database and query for the next best view the patient needs to scanned so as to capture information of certain aspect of the organ.

Internally, it is preferred that the database gets information of the list of scans which it previously suggested and also gets information of the set of scans that were performed. Apart from this information, the information from the images or image-like datasets is used to query the database for the optimal view to detect a particular landmark. An example of the query could be "Patient has frozen shoulder and mastectomy on left side and is difficult to position for MLO view. Suggest an alternative?".

Continuously updating the database is preferred in order to enhance the view-lists. The database is very advantageous prior to the scan and during the scan so as to ensure good quality scan and minimal recalls. Post acquiring the images, the scans (medical volumes) are preferably reviewed by the medical expert/radiologist. If the radiologist can perform diagnosis based on the presented scans, then the database is updated with the views the patient was scanned, the set of landmarks detected in each scan and the logged clinical parameters. This ensures that in the future when a similar patient is scanned, then optimized view-lists could be provided such that the region of interest can be visualized which minimal number of scans. Additionally, to ensure dosage reduction, the system is preferably designed to rank the view chosen to scan a patient. This is preferably achieved using
a) information obtained from the scans and
b) information mentioned in the examination report, e.g. a mammography report.

The ranking of views is preferably based on the coverage of this view, i.e. how much advantageous information the image adds to the examination result. For example, an "Elevated Cranial-Caudal or Pushed-Up" view is suggested for central and medial aspects of an organ. However, such a view may not encompass the required aspects of the organ for patients with a particular body habitus or patients with a particular skeletal deformation. Under such a scenario, the technologist may resort to other views such as "Caudal-Cranial" view to capture the required aspects. The system preferably captures this knowledge of the technologist and ensures that in the future when a patient with similar physical traits is scanned, it recommends "Caudal-Cranial" over "Elevated Cranial-Caudal" view. This mechanism minimizes the patients from unnecessary exposures.

As part of clinical practice, it is required to present all the acquired images, e.g. mammograms, to a radiologist. Additionally, if the technician acquires additional images due to challenges raised by the patient, it is mandated to mention the reason for these. Typically, the radiological report also contains this information. A Natural Language Processing (NLP) unit could be used to extract this information. The extracted information is then used to enhance the system. Especially, additional Information relating to any challenges mentioned by the technician which lead to capturing additional views could be used to update the system.

A medical imaging system according to the invention is especially an mammography-system, a tomosynthesis system, an MRI-system or a CT-system or a fluoroscopy-system, and comprises the system according to the invention.

Some units or modules of the invention mentioned above can be completely or partially realized as software modules running on a processor of a computing system. A realization largely in the form of software modules can have the advantage that applications already installed on an existing computing system can be updated, with relatively little effort, to install and run these units of the present application. The object of the invention is also achieved by a computer program product with a computer program that is directly loadable into the memory of a computing system, and which comprises program units to perform the steps of the methods, at least those steps that could be executed by a computer, when the program is executed by the computing system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a computing system. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

According to a preferred method the physical condition of the patient is estimated in an optical image and/or image-like dataset with the steps:
- detecting the patient in an optical image and/or image-like dataset and
- detecting keypoints of the patient and/or an anatomical location of the patient where the medical examination is planned,
- searching in the area given by the keypoints and/or the anatomical location for indicators of the physical conditions of the patient, and preferably also in other predefined areas of the patient that could have an influence on the examination, especially searching for skeletal malformations and/or physical limitations of the patient, (e.g. for a broken arm or leg).

To detect a patient in an optical image and/or image-like dataset is state of the art. The image could be segmented by a trained Al-model or by a conventional algorithm, at least when the usual position of the patient is known. If a depth information is provided, the nearest pixels may be used as a representation of the patient (and the floor could be deleted).

When the patient is detected, it is easy to detect keypoints of the patient or an anatomical location of the patient where the medical examination is planned. This is also known in the art.

Searching for indicators of the physical conditions of the patient could be performed by measuring the distances of the detected keypoints or by further segmenting the pixels recognized as patient e.g. for a wheelchair or injuries. Here also other predefined areas of the patient could be examined, for example, when the breast is the region of interest, then it could be examined whether the patient has a broken arm or sits in a wheelchair.

It is preferred that additional non-image-data comprising information about the patient is used, especially one or more items of the group comprising, sex, age, weight, measures, physical limitations (this could e.g. be a broken arm but also a single side mastectomy) and disabilities.

According to a preferred method, the view-list is generated by querying a database with a query based on the estimated physical condition of the patient and the requested medical imaging procedure, wherein the database comprises datasets of individual lists of optimal views for multiple different physical conditions for at least one medical imaging procedure. Preferably, the retrieved data comprises the distinct views that are recommended to scan the region of interest. For example, for the whole breast, this could be an elevated cranio caudal view instead of a cranio caudal view.

It is preferred that at least some of the datasets also comprise information of key indicators, such as the length of Posterior Nipple Line in each mammogram could be highlighted to the technologist in order to check the image quality after the acquisition. Alternatively or additionally at least some of the datasets could also comprise information about positioning guidance for a suggested view and/or information of a set of landmarks visible in each view.

A preferred method comprises the additional steps:
- acquiring images from the suggested views in the course of the medical imaging procedure, and preferably outputting the acquired images, especially graphically displaying the coverage of the breast tissue, especially as each image is taken,
- assessing, whether the acquired images are sufficient to fulfil the request and if not:
- recommending additional views considering the patient condition,
while or after acquiring images from the suggested views in the course of the medical imaging procedure.

According to a preferred method, for recommending additional views a database (the same or a different one) is queried for a number of next best views based on information of the view-list which was previously recommended and also gets information of the set of views for that have already been acquired an image. Preferably, images already recorded based on the view-list are examined, whether they fulfil their task or not. In the case an image does not fulfil its task, an alternative view is suggested by the system that is preferably retrieved from the database. It is preferred that, based on the estimated physical condition or an added physical condition of the patient, the database is additionally queried for an optimal view to detect a particular landmark. An example of the query could be: "Patient has frozen shoulder and mastectomy on left side and is difficult to position for MLO view. Suggest an alternative?". This could be solved by including an Al-model in the system that is trained to output views based on the words of an inputted query.

According to a preferred method, images acquired from the recommended views are reviewed by a machine learning model trained to perform a diagnosis based on the images or by a medical expert, e.g. a radiologist, and the database is updated with the views for which images have been acquired in the course of the medical imaging procedure. It is preferred that the database is additionally updated with a number of landmarks detected in each image and/or logged clinical parameters, and/or changed physical condition of a patient, e.g. additional limitations impacting positioning. The system is preferably also updated with additional views and/or links to special views from certain physical conditions of the patient. This ensures that in the future when a similar patient is scanned, then enhanced views are presented in the view-list that are optimized for optimal imaging of an organ under special physical conditions of a patient.

It is preferred that, a report of the imaging procedure is processed by a Natural-Language-Processing-Unit trained for searching for information of a number of additional views that have been recommended in the report, extract this information and update the database and preferably also the system with the extracted information. Preferably, this system comprises this Natural-Language-Processing-Unit.

According to a preferred system the estimation-unit comprises:
- a detection-model being a machine learning model trained to detect the physical condition of the patient,
- a deviation-model being a machine learning model trained to detect physical deviations of the patient from a reference condition based on the output of the detection-model (e.g. skeletal malformations such as kyphosis or scoliosis).

It is preferred that the detection-model is trained to:
a) detect the patient and
b) detect keypoints of the patient and/or an anatomical location of the patient where the medical examination is planned, and preferably also in other areas of the patient that could have an influence on the examination, especially searching for skeletal malformations and/or physical limitations of the patient.

It is preferred that the detection-model is trained to also process non-image data comprising information about the patient, especially one or more items of the group comprising, age, weight, size, inabilities, physical limitations. A such trained model may use the additional information of the non-image data to process the images. Alternatively or additionally it is preferred that the deviation-model is trained to detect skeletal malformations of a patient, especially kyphosis or scoliosis, and/or physical limitations, especially attached wires or IV lines, wheelchair bound patients, patients with feeding tubes or bed ridden patients.

It is preferred that the detection-model is trained to also process non-image data comprising information about the patient, especially one or more items of the group comprising, age, weight, size, inabilities and/or physical limitations.

The methods may also include elements of "cloud computing". In the technical field of "cloud computing", an IT infrastructure is provided over a data-network, storage space or processing power and/or application software. The communication between the user and the "cloud" is achieved by means of data interfaces and/or data transmission protocols. In the context of "cloud computing", in a preferred embodiment of the methods according to the invention, provision of data via a data channel (for example a data-network) to a "cloud" takes place. This "cloud" includes a (remote) computing system, e.g. a computer cluster that typically does not include the user's local machine. It is particularly preferred that the cloud service provides as well computing power as application software.

Other objects and features of the present invention will become apparent from the following detailed descriptions considered in conjunction with the accompanying drawings. It is to be understood, however, that the drawings are designed solely for the purposes of illustration and not as a definition of the limits of the invention.
- Figure 1: shows a tomosynthesis-system comprising a system according to the invention,
- Figure 2: shows a block diagram of the method according to the invention,
- Figure 3: shows a possible use of the invention for diagnosis,
- Figure 4: outlines the method according to the invention,
- Figure 5: shows an automated review of a report.

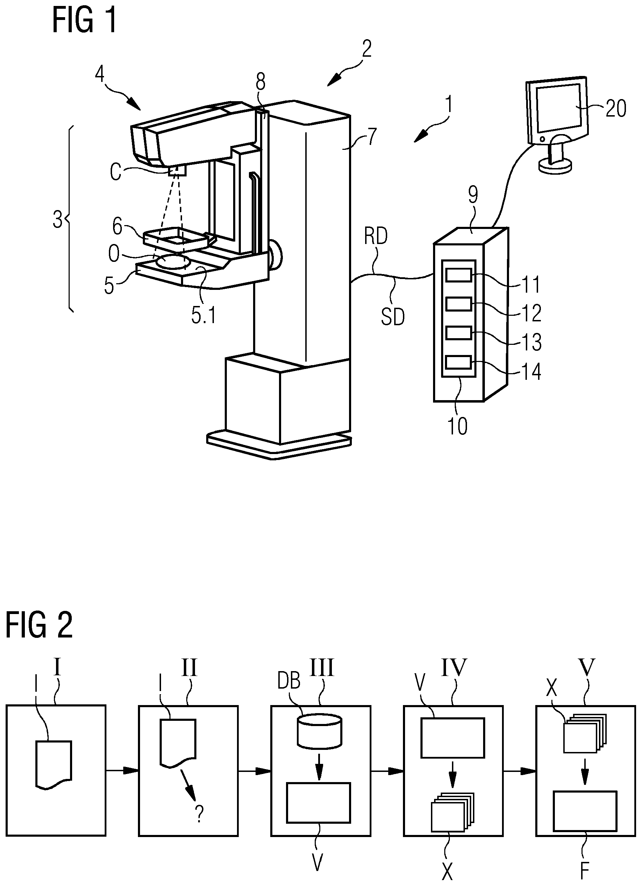

In Figure 1, a mammography system 1 in the form of a tomosynthesis system 1 is roughly schematically shown as an example for a medical imaging system. Relative directional information such as "top", "bottom" etc. refers to a tomosynthesis system 1 that has been set up for operation as intended. The tomosynthesis system 1 includes a tomosynthesis device 2 and a control device 9.

The tomosynthesis device 2 has a column 7 and a source-detector arrangement 3, which in turn include an X-ray emitter 4 and a detector 5 with a detector surface 5.1. The column 7 stands on the ground during operation. The source-detector arrangement 3 is slidably connected to it, so that the height of the detector surface 5.1, i.e. the distance to the ground, can be adjusted to a patient's chest height.

A breast O of the patient (shown here schematically) lies on the top side of the detector surface 5.1 as an examination object O to be examined. A compression plate 6 is arranged above the breast O and the detector surface 5.1, which is slidably connected to the source-detector arrangement 3. For the examination, the breast O is compressed and at the same time fixed by lowering the compression plate 6 onto it, so that pressure is exerted on the breast O between the compression plate 6 and the detector surface 5.1.

The X-ray emitter 4 is arranged and designed opposite the detector 5 in such a way that the detector 5 detects X-ray radiation R emitted by it after at least part of the X-ray radiation R has penetrated the patient's breast O. The X-ray emitter 4 can be pivoted relative to the detector 5 by means of a rotating arm 8 in a certain range about a basic position in which it is vertically above the detector surface 5.1. The section to be recorded can be specified or restricted using a collimator C.

The control device 9 receives the raw data RD of the measurement and sends control data SD to the tomosynthesis device 2 using a data interface. It is connected to a terminal 20 through which a user can communicate commands to the tomosynthesis system 1 or retrieve measurement results. The control device 9 can be arranged in the same room as the tomosynthesis device 2, but it can also be located in an adjacent control room or at an even further spatial distance

The control device 9 comprises a system 10 for providing positioning guidance for medical imaging of a patient, especially for mammography, after a request for a medical imaging procedure. The system 10 comprises an optical measurement unit 11, an estimation-unit 12, a view-unit 13 and a data-interface 14.

The optical measurement unit 11 is designed to acquire optical images X or image-like datasets of a patient. This optical measurement unit 11 may be a LIDAR system designed to measure the surface of a patient, a depth-camera designed for acquiring depth images X, a stereo-camera designed for acquiring multiple images X and calculating a 3D-image X, a TOF-camera designed for acquiring 3D-images X with time of flight information, a triangulation system designed to calculate a surface map of a patient based on distortions of a mesh projected on the patient and optically recorded or a light field camera, designed to calculate a 3D image X from light field data.

The estimation-unit 12 is designed for automatically estimating the physical condition of the patient based on the content of the acquired number of images X and/or image-like datasets.

The view-unit 13 is designed for generating a view-list V comprising a number of suggested optimal views for the requested medical imaging procedure based on the estimated physical condition of the patient.

The data-interface 14 is designed for outputting the generated view-list V for image acquisition.

Figure 2 shows a block diagram of the method according to the invention for providing positioning guidance for medical imaging of a patient, e.g. for mammography.

In step I, a number of optical images I of the patient are acquired.

In step II, a physical condition of the patient based on the content of the acquired number of images I is automatically estimated. The physical condition of the patient could be estimated b detecting the patient in an optical image I, detecting keypoints K of the patient and/or an anatomical location of the patient where the medical examination is planned and searching in the area given by the keypoints K and/or the anatomical location for indicators of the physical conditions of the patient, and preferably also in other predefined areas of the patient that could have an influence on the examination, especially searching for skeletal malformations and/or physical limitations of the patient.

In step III, a view-list V comprising a number of suggested optimal views is generated for the requested medical imaging procedure based on the estimated physical condition of the patient. The view-list V is generated by querying a database DB with a query based on the estimated physical condition of the patient and the requested medical imaging procedure, wherein the database DB comprises datasets of individual views for multiple different physical conditions for at least one medical imaging procedure.

In step IV, images X are acquired based on the view-list V.

In step V, the acquired images X are examined in order to create feedback-data F. This may be achieved by assessing, whether the acquired images X are sufficient to fulfil the request and if not recommending additional views considering the patient condition. For recommending additional views the database DB used in step III could be queried for a number of next best views based on information of the list of scans which were previously recommended. Then, again images X could be acquired from the recommended views.

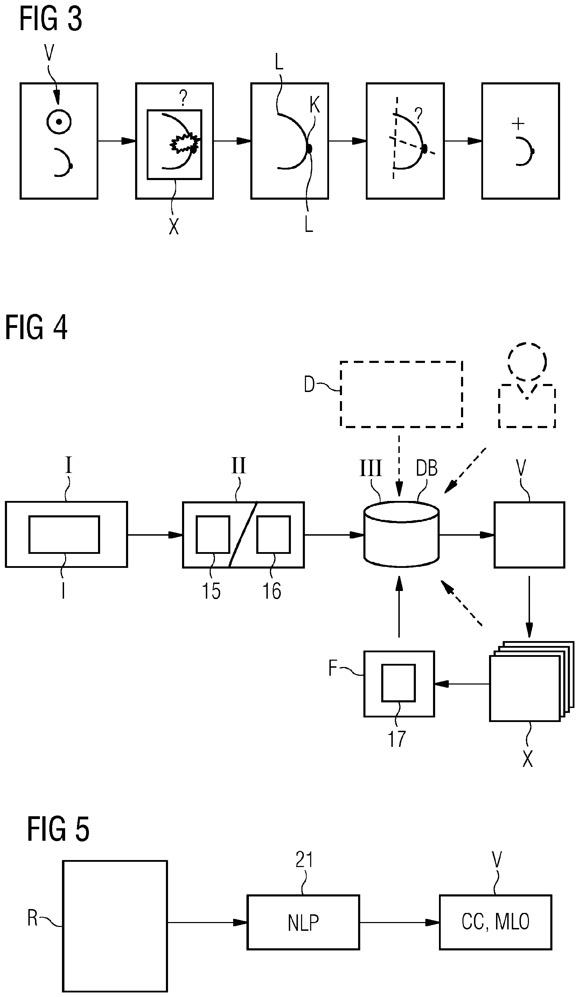

Figure 3 shows a possible use of the invention for diagnosis. From left to right there is the view-list V showing the desired views for a mammography, a recorded view (image X) that is checked for artifacts that may affect the determination of landmarks, and an image where landmarks L are detected, here the Nipple (also keypoint K) and the Pectoralis Muscle. Then values, e.g. the PNL are derived from these landmarks and it is checked, whether these values lie within an acceptable range. In the last picture, an additional view is suggested, in order to have all landmarks visible for the examination.

Figure 4 outlines the method according to the invention. In step I, optical images I are acquired. In this example, the estimation-unit 12 of the system 10 comprises a detection-model 15 being a machine learning model trained to detect the physical condition of the patient and a deviation-model 16 being a machine learning model trained to detect physical deviations of the patient from a reference condition based on the output of the detection-model 15. In this example, the detection-model 15 is trained to detect the patient and detect keypoints K of the patient and/or an anatomical location of the patient where the medical examination is planned, and preferably also in other areas of the patient that could have an influence on the examination, especially searching for skeletal malformations and/or physical limitations of the patient. The detection-model 15 could also be trained to also process non-image data D comprising information about the patient, especially one or more items of the group comprising, age, weight, size, inabilities, physical limitations.

The deviation-model 16 is trained to detect skeletal malformations of a patient, for instance kyphosis or scoliosis, and/or physical limitations, especially attached wires or IV lines, wheelchair bound patients, patients with feeding tubes or bed ridden patients. In this example, the detection-model 15 is trained to also process non-image data D comprising information about the patient, especially one or more items of the group comprising, age, weight, size, inabilities, physical limitations.

The view-list V is retrieved from a database DB, wherein non-image data D and expert knowledge (dashed) could also be used. The view-list V is then used to acquire images X for the examination (on the right).

The system 10 also comprises an assessment-unit 17 designed for assessing, whether the acquired images X are sufficient to fulfil the request. The assessment-unit 17 comprises an algorithm, especially a trained machine-learning model, designed for checking the acquired images X whether a set of predetermined landmarks L can be determined from the images X (see figure 3). In this example, the assessment-unit 17 is further designed for choosing additional views based on the assessment that are able to show missing regions of the acquired images X and outputting these additional views. These additional views are retrieved from the database DB and used to acquire additional images X.

The dashed arrow from the acquired images X to the database DB should indicate that an expert could examine the images and acquire additional views manually.

Figure 5 shows an automated review of a report of the imaging procedure. It is processed by a Natural-Language-Processing-Unit 21 trained for searching for information of a number of additional views that have been recommended in the report, extract this information and add the views to a view-list V. wherein the database is updated with the extracted information.

Although the present invention has been disclosed in the form of preferred embodiments and variations thereon, it will be understood that numerous additional modifications and variations could be made thereto without departing from the scope of the invention. For the sake of clarity, it is to be understood that the use of "a" or "an" throughout this application does not exclude a plurality, and "comprising" does not exclude other steps or elements. The expression "a number of" means "at least one". The mention of a "unit" or a "device" does not preclude the use of more than one unit or device. Independent of the grammatical term usage, individuals with male, female or other gender identities are included within the term.

## Claims

1. A method for providing positioning guidance for medical imaging of a patient, especially for mammography, after a request for a medical imaging procedure, comprising the steps:
a) acquiring a number of optical images (I) and/or image-like datasets of the patient,
b) automatically estimating a physical condition of the patient based on the content of the acquired number of images (I),
c) generating a view-list (V) comprising a number of suggested optimal views for the requested medical imaging procedure based on the estimated physical condition of the patient,
d) outputting the generated view-list (V) for image acquisition.

2. The method according to claim 1, wherein the physical condition of the patient is estimated in an optical image (I) and/or image-like dataset with the steps:
- detecting the patient in an optical image (I) and/or image-like dataset and
- detecting keypoints (K) of the patient and/or an anatomical location of the patient where the medical examination is planned,
- searching in the area given by the keypoints (K) and/or the anatomical location for indicators of the physical conditions of the patient, and preferably also in other predefined areas of the patient that could have an influence on the examination, especially searching for skeletal malformations and/or physical limitations of the patient,
preferably wherein additional non-image data (D) comprising information about the patient is used, especially one or more items of the group comprising, sex, age, weight, measures, physical limitations and disabilities.

3. The method according to one of the preceding claims, wherein the view-list (V) is generated by querying a database with a query based on the estimated physical condition of the patient and the requested medical imaging procedure, wherein the database (DB) comprises datasets of individual lists of optimal views for multiple different physical conditions for at least one medical imaging procedure,
preferably wherein at least some of the datasets also comprise information of key indicators and/or information about positioning guidance for a suggested view or of a set of landmarks (L) visible in each view.

4. The method according to one of the preceding claims, comprising the additional steps:
- acquiring images (X) from the suggested views in the course of the medical imaging procedure,
- assessing, whether the acquired images (X) are sufficient to fulfil the request
and if not:
- recommending additional views considering the patient condition,
while or after acquiring images (X) from the suggested views in the course of the medical imaging procedure.

5. The method according to claim 4, wherein for recommending additional views a database (DB) is queried for a number of next best views based on information of the list of scans which were previously recommended and also gets information of the set of views for that have already been acquired an image (X),
preferably wherein, based on the estimated physical condition or an added physical condition of the patient, the database (DB) is additionally queried for an optimal view to detect a particular landmark (L).

6. The method according to one of the preceding claims, wherein images (X) acquired from the recommended views are reviewed by a machine learning model trained to perform a diagnosis based on the images (X) or by a medical expert, and the database (DB) is updated with the views for which images (X) have been acquired in the course of the medical imaging procedure, preferably wherein the database (DB) is additionally updated with a number of landmarks (L) detected in each image (X) and/or logged clinical parameters, and/or changed physical condition of a patient,
preferably wherein, a report (R) of the imaging procedure is processed by a Natural-Language-Processing-Unit (21) trained for searching for information of a number of additional views that have been recommended in the report (R), extract this information and update a database (DB) and/or the system (10) with the extracted information.

7. A system (10) for providing positioning guidance for medical imaging of a patient, especially for mammography, after a request for a medical imaging procedure, comprising:
- an optical measurement unit (11), designed to acquire optical images (I) or image-like datasets of a patient,
- an estimation-unit (12) designed for automatically estimating the physical condition of the patient based on the content of the acquired number of images (I) and/or image-like datasets,
- a view-unit (13) designed for generating a view-list (V) comprising a number of suggested optimal views for the requested medical imaging procedure based on the estimated physical condition of the patient,
- a data-interface (14) designed for outputting the generated view-list (V) for image acquisition.

8. The system (10) according to claim 7, wherein the estimation-unit (12) comprises:
- a detection-model (15) being a machine learning model trained to detect the physical condition of the patient,
- a deviation-model (16) being a machine learning model trained to detect physical deviations of the patient from a reference condition based on the output of the detection-model (15),
preferably wherein the detection-model (15) is trained to:
a) detect the patient and
b) detect keypoints (K) of the patient and/or an anatomical location of the patient where the medical examination is planned, and preferably also in other areas of the patient that could have an influence on the examination, especially searching for skeletal malformations and/or physical limitations of the patient,
preferably wherein the detection-model (15) is trained to also process non-image data (D) comprising information about the patient, especially one or more items of the group comprising, age, weight, size, inabilities, physical limitations.
and/or
preferably wherein the deviation-model (16) is trained to detect skeletal malformations of a patient, especially kyphosis or scoliosis, and/or physical limitations, especially attached wires or IV lines, wheelchair bound patients, patients with feeding tubes or bed ridden patients, preferably wherein the detection-model (15) is trained to also process non-image data (D) comprising information about the patient, especially one or more items of the group comprising, age, weight, size, inabilities, physical limitations.

9. The system (10) according to one of claims 7 or 8, comprising an assessment-unit (17) designed for assessing, whether the acquired images (X) are sufficient to fulfil the request, preferably wherein the assessment-unit (17) comprises an algorithm, especially a trained machine-learning model, designed for checking the acquired images (X) whether a set of predetermined landmarks (L) can be determined from the images (X),
preferably wherein the assessment-unit (17) comprises an algorithm, especially a trained machine-learning model, designed for deriving medical parameters from the acquired images (X) and checking whether these parameters fall within a predefined range,
preferably wherein the assessment-unit (17) is further designed for choosing additional views based on the assessment that are able to show missing regions of the acquired images (X) and outputting these additional views.

10. The system (10) according to one of claims 7 to 9, comprising a number of machine-learning models trained for one or more of the tasks of the group comprising
- determining a view shown in an image (X),
- detecting medical landmarks (L) in an image
- segmenting medical objects in an image (X),
- inspecting an image (X) for artifacts.

11. The system (10) according to one of claims 7 to 10, wherein the optical measurement unit (11) is
- a LIDAR system designed to measure the surface of a patient,
- a depth-camera designed for acquiring depth images (I),
- a stereo-camera designed for acquiring multiple images (I) and calculating a 3D-image (I),
- a TOF-camera designed for acquiring 3D-images (I) with time of flight information,
- a triangulation system designed to calculate a surface map of a patient based on distortions of a mesh projected on the patient and optically recorded,
- a light field camera, designed to calculate a 3D image (I) from light field data.

12. The system (10) according to one of claims 7 to 11, comprising a user interface designed to display the acquired images (X) to a user and to receive feedback (F) of the user,
wherein the system (10) is preferably designed to use this feedback (F) in combination with the data inputted and generated in the course of the medical imaging procedure to further train machine learning models of the system.

13. A medical imaging system, especially a mammography-system (1), a tomosynthesis system, an MRI-system or a CT-system or a fluoroscopy-system, comprising the system (10) according to one or more of claims 7 to 12.

14. A computer program product comprising instructions which, when the program is executed by a computer, cause the computer to carry out the steps of the training-method of any of claims 1 to 6.

15. A computer-readable storage medium comprising instructions which, when executed by a computer, cause the computer to carry out the steps of the training-method of any of claims 1 to 6.
